# EUROPEAN PATENT APPLICATION

(11) **EP 2 668 914 A1**
(43) Date of publication of application: **04.12.2013**
(21) Application number: 12181590.6
(22) Date of filing: 23.08.2012
(51) Int. Cl.: A61B 17/12

(54) **Implant system**

(30) Priority: 01.06.2012 EP 12170456
(71) Applicant: Acandis GmbH & Co. KG, 76327 Pfinztal (DE)
(72) Inventor: Watson, David, San Jose, CA 95125 (US)
(74) Representative: Kilchert, Jochen

(57) **Abstract**

Provided is an implant system (10) for occlusion of vascular anomalies comprising an elongated delivery means (20) and a medical device (30) for release within the vasculature of a patient wherein the elongated delivery means has proximal and distal ends and at least a first electrical conducting means (21) and wherein said elongated delivery means includes an insulated conductive transport wire (25) as first electrical conducting means. The proximal end of the medical device is formed from the distal end of the transport wire and the insulated conductive transport wire terminates as a non-insulated sacrificial segment (26) proximal to the medical device. Further, the medical device is releasable from the elongated delivery means by applying electrical power to the first electrical conducting means such that current flows through the said sacrificial segment to an electrical grounding means to electrolytically degrade said sacrificial element releasing the medical device. Thus, the present invention has the advantage that the proximal end of the medical device is formed from the distal end of the transport wire which thus forms an element of an articulating junction. The present invention also provides a method for releasing a medical device within the vasculature of a patient using the implant system.

## Description

### Field of the Invention

The invention relates to the field of vaso-occlusive devices for the treatment of various vascular anomalies such as for example aneurysms, arterio-venous fistula, and artery occlusions. More particularly, the invention provides an implant system for occlusion of vascular anomalies that includes an elongated delivery means and a releasable medical device that after deployment conforms to the shape of an anatomical cavity. Further, the invention relates to a method of releasing such a medical device.

### Background of the Invention

The treatment of vascular anomalies such as aneurysms, arterio-venous fistula, and artery occlusions or other vascular anomalies remains an important area of research both to physicians and patients alike and has improved dramatically in recent years by placement of devices within a vasculature of a patient such that many vascular anomalies are treated in-situ.

In general, vaso-occlusion devices are therapeutic devices that are placed within the vasculature of the human body, typically via a catheter, either to block the flow of blood through a vessel making up that portion of the vasculature through the formation of an embolus or to form such an embolus within an aneurysm stemming from the vessel, thus ultimately healing the aneurysm.

Moreover, such devices allow a physician to treat a patient non-invasively so that exposure to risk factors associated with invasive surgery is minimized. That is, the placement of vaso-occlusive devices within a vasculature of a patient allows the physician to treat a patient without using surgical techniques that are considered high risk to the patient.

These vaso-occlusive devices can be produced in such a way that they will pass through the lumen of a catheter in a linear shape and take on a complex shape as originally formed after being deployed into the area of interest, such as an aneurysm. These devices are commonly produced as primary coiled winding of an implantable metal that is subsequently formed into a more complex secondary shape by winding the primary winding about poles placed on a winding mandrel. The secondary wound coil is then annealed on the winding mandrel, and the coil is then removed from the winding mandrel. One widely used vaso-occlusive device as described in US patent 5,645,558 is a helical wire coil having a deployed configuration which is generally spherical shaped and can be dimensioned to engage the walls of the vessels or aneurysm. The delivery of such vaso-occlusive devices can be accomplished by a variety of means, including via a catheter in which the device is pushed through the catheter by a pusher to deploy the device. The embolic coil is releasably attached to a pusher member such that the physician may manipulate the position of the coil prior to detaching it in-situ. Various detachment mechanisms to release the device from a pusher have been developed and are known in the art.

For treatment of areas of the small diameter vasculature such as a small artery or vein in the brain, for example, and for treatment of aneurysms and the like, micro-coils formed of very small diameter wire are used in order to restrict, reinforce, or to occlude such small diameter areas of the vasculature. The primary winding of these micro-coils is generally formed from a very small metallic wire with a diameter generally ranging from 40 to 80 micrometers. This wire is formed into a primary coil generally about 0.25mm - 0.50mm in diameter such that it can be inserted in a linear configuration up a correspondingly sized microcatheter. A variety of materials have been suggested for use in such micro-coils, including nickel-titanium alloys, copper, stainless steel, platinum, tungsten, various plastics or the like, each of which offers certain benefits in various applications. Platinum alloys are particularly advantageous for the fabrication of such micro coils, in that they can have suitable shape memory properties, radiopacity to be seen with x-ray during deployment, and can be manufactured to easily fit into a linear portion of a catheter, yet attain their originally formed, more complex shape when deployed.

One described vaso-occlusive coil is known from US 6,638,291, for example, that has a three dimensional in-filling coil configuration, formed by winding a wire into a primary helix, and then winding the primary helix into a secondary form which forms a generally spherical shape, by winding the primary coil about poles placed on winding mandrel. The secondary wound coil is then annealed on the winding mandrel, and the coil is then removed from the winding mandrel and loaded into a carrier for introduction into a delivery catheter. These widely used vaso-occlusive devices are generally constructed from a primary coil that is wound from a single wire of uniform material and most commonly is a Platinum alloy wire. As Platinum alloys are precious metal, the cost of such coils is generally quite high. Also, since numerous coils are often needed to treat a particular vessel, the density of the Platinum in the vessel creates an imaging artifact that is often undesirable.

Yet another conventional implant for vessel occlusion is made from helical elements of metal or synthetic material by twisting or coiling the elements and forming them into a secondary shape such as a rosette or double rosette for implantation using a catheter. In particular, US Patent US 5,733,329 discloses a vaso-occlusive that has a final conical shape. However, due to the tendency of such three dimensional shaped coils to transform into their expanded, final forms when introduced into a catheter in the body, they are inherently more difficult than a helical coil or a straight wire or micro-cable to push through such a catheter for delivery to a site in vasculature to be treated, due to friction between the coil and the catheter through which it is delivered to the site to be treated, which can even result in misalignment of the coil within the catheter during delivery.

Another apparatus that is known is disclosed in US 6,478,773. In particular, such an apparatus deploys a therapeutic device such as a micro-coil which is detachably mounted to a distal portion of a pusher member. In particular, the therapeutic device is detachably mounted to the distal portion of the pusher member by a tubular collar that can be heated by a heater such as an electrical resistance coil to expand the collar and release and deploy the therapeutic device. Moreover, the apparatus for deployment of a therapeutic device such as a micro-coil also provides a pusher member and a connector fiber for securing the therapeutic device to the pusher member. The connector fiber passes through a heater within the distal portion of the pusher member, for heating and breaking the connector fiber to release the therapeutic device when a desired placement of the therapeutic device within the vasculature is achieved. However, such an apparatus is complex in nature since at least it requires a heater to sever the fibre.

In yet another publication, European Patent 1,985,240, a delivery system for delivering an implantable medical device to a target location of a body vessel is described. The system comprises a generally hollow tubular carrier member which defines a longitudinal axis and having a proximal portion, which is adapted to remain outside the body when in use, a distal portion, at least a portion of which is adapted to be positioned inside the body when in use, and a frangible portion intermediate said proximal portion and said distal portion. The frangible portion comprises at least one notch providing a break hinge transverse to said longitudinal axis of the carrier member. However, such a system relying on the frangible portion to separate the proximal and distal portion may inadvertently break during placement and manipulation in a body vessel.

In another publication, US 5,423,829, a guidewire for use in the formation of a vascular occlusion, in combination with a catheter is described and which comprises a core wire. The core wire has an axis and not being susceptible to electrolytic disintegration in blood, a discrete, sacrificial, severable link susceptible to electrolytic disintegration in blood distal to and severably connected to the core wire. Furthermore, an elongate tip portion extends distally beyond the core wire and adapted to form the occlusion at a selected site within a mammal vasculature. The elongate tip portion is not susceptible to electrolytic disintegration in blood and severable from the core wire upon electrolytic disintegration of the sacrificial link. However, such an arrangement provides a relatively complicated solution and thus costly to manufacture.

In yet another publication, US 5,984,929, an implant assembly for placement of an implant in the human body is described and comprises an implant member having a proximal end and a distal end, and a wire having an electrolytically severable joint. The wire is attached to the implant member proximal end, and the electrolytically severable joint being relatively more susceptible to electrolysis in an ionic solution than the implant member. The implant member is electrically isolated from said electrolytically severable joint by a continuous, insulative or highly resistive layer disposed on said wire proximal of the joint and a continuous, insulative or highly resistive layer disposed on said wire distal of the joint. However, such an arrangement may be difficult manipulate with the mammalian vasculature and thus damage the interior wall of the vasculature.

From the foregoing, it can be seen that vaso-occlusive devices have provided important improvements in occlusion devices for the treatment of aneurysms and various types of arteriovenous anomalies, but there remains important limitations in the technology presently available.

It is an object of the present invention to provide an implant system comprising a medical device that is improved as regards to the handling by a physician during delivery to a target site. Moreover, it is an object of the present invention to provide a method for releasing a medical device of an implant system.

### Summary of the Invention

This object is achieved by the implant system of claim 1, alternatively by the system of claim 5. With regards to the method, said object is achieved by the subject matter of claim 8.

The present invention provides an implant system for occlusion of vascular anomalies comprising an elongated delivery means and a medical device for release within the vasculature of a patient wherein the elongated delivery means has proximal and distal ends and at least a first electrical conducting means and wherein said elongated delivery means includes an insulated conductive transport wire as first electrical conducting means. The proximal end of the medical device is formed as a loop segment from the distal end of the transport wire and the insulated conductive transport wire terminates as a non-insulated sacrificial segment proximal to the medical device. Further, the medical device is releasable from the elongated delivery means by applying electrical power to the first electrical conducting means such that current flows through the said sacrificial segment to an electrical grounding means to electrolytically degrade said sacrificial element releasing the medical device.

The present invention is based on the idea that the distal end of the delivery means remains at the implanted medical device. The advantage of the present invention is the simple configuration and the high flexibility of the connection between the delivery device, in particular the transport wire, and the medical device, in particular a medical coil. Moreover, this configuration allows for a simplified construction of an articulating junction between the delivery means and the medical coil, thus further enhancing the flexibility, in particular with respect to navigating the system through a patient's vasculature.

That is, the non-insulated sacrificial segment is limited by the insulated transport wire proximal of the sacrificial segment and may be further limited by an insulated ring distal of the sacrificial segment. The insulated ring may form part of the elongated delivery means.

According to a preferred embodiment of the present invention the proximal end of the medical device is formed from the distal end of the transport wire which thus may form an element of an articulating junction. Such an arrangement provides the additional advantage of improved manipulation of the medical device to a target site within the vascular area. Moreover, and compared to the prior art, the characteristic articulated joint further provides the advantage of a stretch resistant member to be commonly interconnected at the articulating junction which improves the handling characteristics of the medical device.

Furthermore, the costs associated with implanting the medical device of the present invention to the health care organisations is reduced since the articulating junction provides greater manipulation of the medical device to the target site, thus requiring less time by a physician.

Further preferred embodiments of the invention are indicated in the dependent claims.

In particular, the electrically grounding means is provided by a second electrical conducting means arranged along at least a portion of the elongated delivery means that is electrically insulated from the said first conducting means and terminates approximate the said sacrificial segment. Such an arrangement provides an inexpensive implant system allowing a physician to be void of additional electrical cables.

Moreover, the said second conducting means may be provided by a conductive enclosure at least partially encasing the said first conducting means at least along a portion of the length of the said elongated delivery means. The element forming the articulating junction may be composed of the loop segment interconnected at a position spaced from the sacrificial segment.

The loop segment may be integrally formed from the distal end of the transport wire and the loop segments may be engaged with an attachment loop at the proximal end of the medical device.

According to a co-ordinate aspect, the present invention also provides a system for release of a medical device within the vasculature of a patient, including an elongated delivery means, said medical device, and an articulatable junction there between, wherein said elongated delivery means has a proximal end and a distal end and an at least one first transport wire extending from proximate the said proximal end of the elongated delivery means, and terminating as a loop formed of said transport wire at said distal end, said transport wire provides an insulated electrical conducting means from a terminal contact point proximate to said proximal end of said elongated delivery means and terminating proximal to said formed loop as a non-insulated sacrificial segment of transport wire. The medical device is configured with a proximal hook to be movably affixed through said formed loop forming said articulating junction.

All features of embodiments disclosed in connection with the system of claim 1 are also disclosed in connection with the system of claim 5.

The present invention also provides a method for releasing a medical device using the implant system of claim 1 wherein the sacrificial segment is electrolytically degraded releasing the medical device including the articulating junction. The medical device may be released within the vasculature of a patient. In this case, the medical device may be delivered to the lesion site prior to the degrading the sacrificial segment.

### Brief description of the Drawings

A better understanding of the features and advantages of the present invention will be obtained by reference to the following detailed description that sets forth illustrative embodiments by way of example only, in which the principles of the invention are utilized, and the accompanying drawings of which:
Figure 1 shows a partial cross-sectional view of an embodiment of an implant system according to the present invention and including an elongated delivery means and a medical device.
Figure 2 shows a partial cross-sectional view of a medical device separated from an elongated delivery means of the implant system according to the present invention.
Figure 3 shows a partial cross-section view of another embodiment of an implant system according to the present invention and including a stretch resistant fibre.

### Detail Description of the Invention

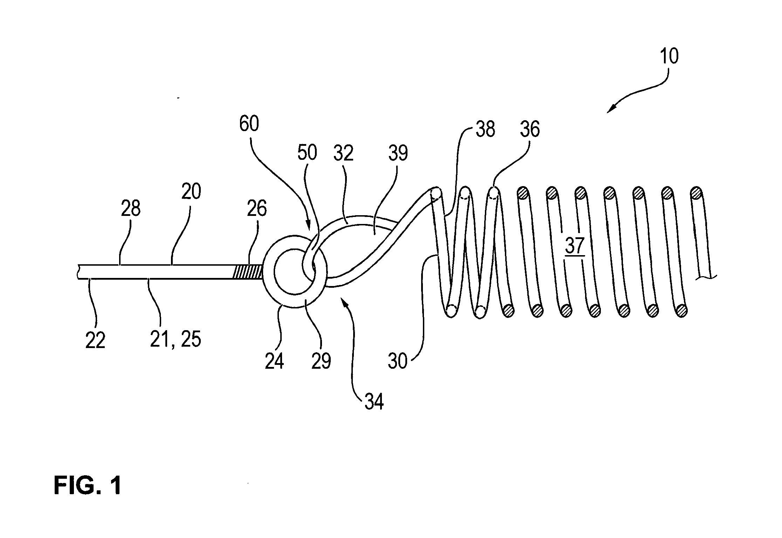

Figure 1 shows a simplified partial cross-sectional view of an embodiment of an implant system 10 according to the present invention. Implant system 10 is shown removed from the vasculature of a patient for the purposes of clarity. Implant system 10 comprises an elongated delivery means 20 such as a pusher and a medical device 30. Elongated delivery means 20 allows a medical practitioner to manipulate medical device 30 within the vasculature of a patient. Elongated delivery means 20 comprises a proximal end 22 and a distal end 24. Elongated delivery means 20 is formed from an electrical conductor such as a wire to provide a first electrical conducting means 21. Electrical conducting means of the elongated delivery means 20 is mostly insulated 28 and may be defined as an insulated conductive transport wire 25. Distal end 24 of transport wire 25 terminates as a closed loop or a closed loop segment 29. The insulated conductive transport wire 25 is however not insulated at the sacrificial segment 26 proximal to the closed loop 29.

Preferably, insulated conductive transport wire 25 is insulated by means of an implantable grade coating. In particular, the electrical conducting means 21 is coated by means of a chemical vapor deposition such as provided by a polymer, for example Paralyene^{™}. Although not shown in the current figure, proximal end 22 of elongated delivery means 20 is connected to a power source, for reasons that will be explained later.

Distal end 24 of transport wire 25, that is end proximal to medical device 30 is preferably formed as a closed loop segment 29. Inner diameter of loop segment 29 is defined by the diameter of an attachment loop 32 of medical device 30 as will be discussed later. In particular however, closed loop segment 29 of transport wire 25 is further insulated by means preferably of chemical vapor deposition to further define the non-insulated sacrificial segment 26.

Further shown in the present figure is medical device 30. Medical device 30 may be generally defined as a flexible element that is formed from a primary winding composed of at least one wire 38 being helically wound to form a helical winding 36. Helical winding 36 may be composed of several wires (not shown) depending on for example the nature and location of the vascular anomaly. Generally, helical winding 36 is wound on a common longitudinal axis such as for example a mandrel (not shown) and made from, for example an implantable grade Pt-W alloy. As shown, internal diameter of helical winding 36 forms a longitudinal lumen 37.

As shown in the current figure, proximal end 34 of medical device 30, in particular helical winding 36, is arranged through closed loop segment 29 of transport wire 25, thus forming a loop 39. That is, such an arrangement provides an engagement portion between elongated delivery means 20 and medical device 30. That is, proximal end 34 of medical device 30 is preferably formed as a closed loop segment 39 that is interconnected with loop 29.

To minimise the risk of inadvertent damage to surrounding vascular walls during and after deployment within a vessel, proximal end 34 of medical device 30 is preferably formed as a stubbed terminal (not shown). Moreover, once proximal end 34 of helical winding 36 is arranged through closed loop segment 29, stubbed terminal of helical winding 36 is either soldered, glued, twisted or a combination thereof to helical winding 36. Such an arrangement provides an additional benefit of securing loops 29 and 39 together. Further, such an arrangement provides an articulating junction (60) between ends 24 and 34. Such an articulating junction (60) allows a physician to manipulate the medical device in the correct position prior to the separation of the medical device 30 from the delivery means 20. As is clearly depicted in the current figure however, the articulating junction (60) composed of interconnected loops 29 and 39 is arranged at a position spaced from sacrificial segment 26. That is, loop 29 is integrally formed from the distal end 24 of the transport wire 25 and engaged with an attachment loop 39 at the proximal end 34 of the medical device.

In one embodiment of the present invention and as shown in the current figure, proximal end 34 of helical winding 36 is only arranged through closed loop segment 29 once. However, it is envisaged, and to provide an articulating joint of different properties, proximal end 34 of helical winding 36 may be arranged multiple times through closed loop segment 29. For example, a physician may elect to use an implant system 10 having an articulating joint that has an increased resistance for a subject that has a decreased level of vascular elasticity as opposed to a subject that has a normal level of vascular elasticity.

In another embodiment (not shown) such a looping arrangement between a delivery means and a medical device allows for the provision for example of several medical devices to be looped from distal end 24. That is, and depending on the characteristics of the vascular anomaly, it may be preferable to insert several medical devices 30 having, optionally, outer diameters that are less than an outer diameter of a single medical device, so that during use the vascular anomaly is treated effectively. Moreover, it is envisaged that the outer surface of one medical device may be of a different nature to a secondary medical device. For example, the outer surfaces of the medical devices may be coated with either a medicament and/or a coagulant.

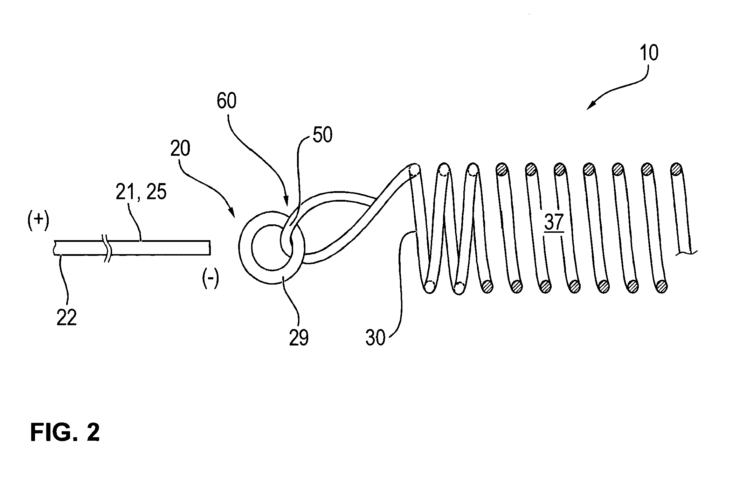

Figure 2 shows a partial cross-sectional view of a medical device 30 separated from an elongated delivery means 20 of the implant system 10 according to the present invention.

Although not depicted in the current figure, proximal end 22 of first electrical conducting means 21 of elongated delivery means 20 terminates at a power source which is connected to an electrical ground. Preferably, first electrical conducting means 21 terminates on a positive terminal of the power supply. In one aspect of the present invention, a subject such as a patient (not shown) may be electrically connected to a grounding means that provides a second electrical conducting means 23. For example, the subject may be connected directly to an electrical earth point or alternatively the operating table (not shown) in which the subject is in contact with during a procedure of inserting the implant system of the present invention may in addition or alternatively be connected to an electrical earth point. In another configuration however, the second electrical conducting means 23 may be provided by a conductive enclosure at least partially encasing the first conducting means 21 at least along a portion of the length of the elongated delivery means. In particular, second electrical conducting means 23 is arranged along at least a portion of the elongated delivery means 20 that is electrically insulated from the first conducting means 21 and terminates approximate the sacrificial segment 26. That is, the non-insulated sacrificial segment 26 is limited by the insulated transport wire proximal of the sacrificial segment 26 and the insulated ring distal of sacrificial segment 26. Moreover however, the length of segment 26 determines the rate that such a segment can be sacrificed. Further, such grounding means provides a closed electrical circuit to the positive terminal of the power supply since a common ground is shared.

According to general practice, a physician will access a target lesion within the vasculature of a patient using a flexible catheter that is generally inserted in the femoral artery at the groin (not shown). The inner diameter of said delivery catheter is sized to accept the medical device 30 and delivery means 20. After introducing the medical device 30 into said catheter the physician by means of the elongated delivery means 20 would advance medical device 30 through said catheter and then deploy it outside the distal end of the catheter and then manipulate its final position within the target lesion (not shown). The physician would be guided to manipulate medical device 30 by means of x-rays, CT scans and or MRI scans to ensure proper positioning of medical device 30 in the target location. Further and by means of the power supply, power is transferred to the transport wire 25 wherein the current flows to the grounding means through an electrolyte (the patient's blood) across the exposed sacrificial segment 26 to electrolytically degrade segment 26 thereby releasing medical device 30 from delivery means 20. As is clearly depicted in the current figure, medical device 30 is released from elongated delivery means 20. In particular, loop 29 forming the distal end of elongated delivery means 20 is released from delivery means 20 and thus articulating junction (60) then becomes part of medical device 30 by means of loops 29 and 39. Such a configuration allows for example the ends i.e. the proximal ends of several helical windings and or other elements (as will be discussed later) to be commonly interconnected at articulating junction (60) which forms part of medical device 30 after segment 26 is sacrificed. Preferably, sacrificed segment 26 leaves a non-traumatic area on loop 29 such that the risk of inadvertent damage to surrounding vascular walls and after deployment within a vascular region is avoided.

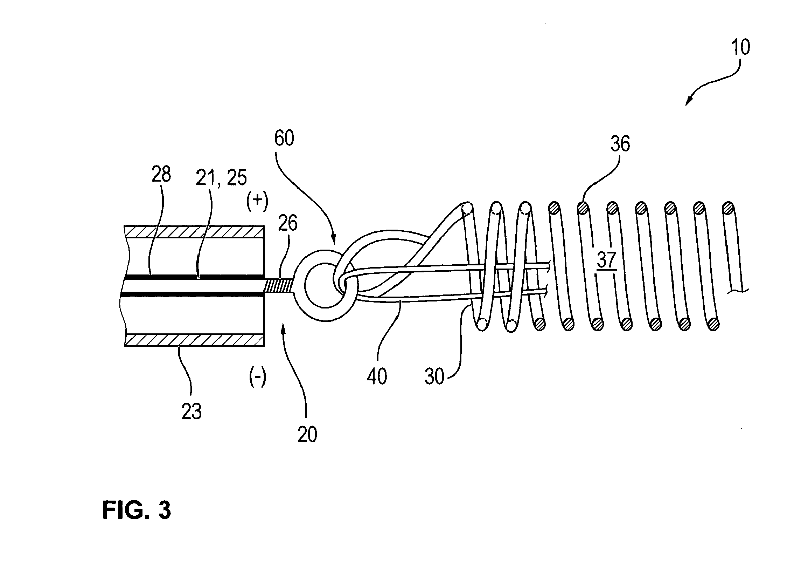

Figure 3 shows a partial cross-section view of another embodiment of an implant system according to the present invention and includes an optional linear elongation limiter such as a stretch resistant fibre 40 which limits the elongation of the medical device 30 during manipulation by the physician. As depicted in the current figure, stretch resistant fibre 40 is additionally arranged through loop 29 and extends along lumen 37 of helical winding 36. Although not depicted in the current figure, optional stretch resistant fibre 40 may extend to a distal end of helical winding 36 and thus being retained thereon by means of gluing, soldering, twisted or a combination thereof. Stretch resistant fibre 40 limits the elongation of medical device 30 prior to release the medical device 30 at its target vascular position and decreases the risk of damage to medical device 30 if for example a significant tensile load is applied thereon. Depending on the characteristic requirement of the medical device, stretch resistant fibre 40 may be arranged though loop 29 a multiple of times. As shown therefore, proximal ends of helical winding 36 and stretch resistant fibre to 40 to be commonly interconnected through loop 29.

Various embodiments of the invention have been described above. The descriptions are intended to be illustrative, not limitative. Thus, it will be apparent to one skilled in the art that certain modifications may be made to the invention as described without departing from the scope of the claims set out below.

## Claims

1. An implant system (10) including an elongated delivery means (20) and a medical device (30) for release within the vasculature of a patient wherein the elongated delivery means (20) has proximal and distal ends (22, 24) and at least a first electrical conducting means (21), wherein
- said elongated delivery means (20) includes an insulated conductive transport wire (25) as first electrical conducting means (21),
- a proximal end (34) of the medical device (30) is formed as a loop segment (29) from the distal end (24) of the transport wire (25) and
- said insulated conductive transport wire (25) terminates as an non-insulated sacrificial segment (26) proximal to the medical device(30);
wherein the medical device (30) is releasable from the elongated delivery means (20) by applying electrical power to the first electrical conducting means (21) such that current flows through the said sacrificial segment (26) to an electrical grounding means to electrolytically degrade said sacrificial element (26) releasing the medical device(30).

2. The implant system according to claim 1 wherein said proximal end (34) of the medical device (30) forms an element of an articulating junction (60).

3. The implant system according to claims 2, wherein the element forming the articulating junction (60) is composed of the loop segment (29) interconnected at a position spaced from the sacrificial segment (26).

4. The implant system according to claim 2 or 3, wherein the loop segment (29) is integrally formed from the distal end (24) of the transport wire (25) and the loop segment (29) is engaged with an attachment loop (39) at the proximal end (34) of the medical device (30).

5. A system for release of a medical device (30) within the vasculature of a patient, including an elongated delivery means (20), said medical device (30), and an articulatable junction (60) there between, wherein;
said elongated delivery means (20) has a proximal end (22) and a distal end (24) and an at least one first transport wire (25) extending from proximate the said proximal end (22) of the elongated delivery means (20), and terminating as a loop (29) formed of said transport wire (25) at said distal end (24), said transport wire (25) provides an insulated electrical conducting means (21) from a terminal contact point proximate to said proximal end of said elongated delivery means (20) and terminating proximal to said formed loop (29) as a non-insulated sacrificial segment (26) of transport wire (25);
said medical device (30) is configured with a proximal hook (39) to be movably affixed through said formed loop (29) forming said articulating junction (60).

6. The implant system according to one of the preceding claims, wherein the electrically grounding means is provided by a second electrical conducting means (23) arranged along at least a portion of the elongated delivery means (20) that is electrically insulated from the said first conducting means (21) and terminates approximate the said sacrificial segment (26).

7. The implant system according to claim 3, wherein the said second conducting means (23) is provided by a conductive enclosure at least partially encasing the said first conducting means (21) at least along a portion of the length of the said elongated delivery means (20).

8. A method for releasing a medical device (30) within the vasculature of a patient using the implant system (10) of claim 1 wherein the medical device (30) is delivered to the lesion site and the sacrificial segment (26) is electrolytically degraded releasing the medical device (30) including the articulating junction.
